(19) Europäisches Patentamt
European Patent Office
Office européen des brevets

(11) Veröffentlichungsnummer: **0 399 284 B1**

(12) # EUROPÄISCHE PATENTSCHRIFT

(45) Veröffentlichungstag der Patentschrift: **02.08.95**

(21) Anmeldenummer: **90108628.0**

(22) Anmeldetag: **08.05.90**

(51) Int. Cl.6: **C07C 22/04**, C07C 25/02, C07C 15/16

(54) **Verfahren zur Herstellung von geminalen Diarylalkanen, neue geminale Diarylalkane und alk(en)ylierte aromatische Verbindungen.**

(30) Priorität: **20.05.89 DE 3916557**

(43) Veröffentlichungstag der Anmeldung:
**28.11.90 Patentblatt 90/48**

(45) Bekanntmachung des Hinweises auf die Patenterteilung:
**02.08.95 Patentblatt 95/31**

(84) Benannte Vertragsstaaten:
**BE CH DE ES FR GB IT LI NL**

(56) Entgegenhaltungen:
**DE-A- 3 916 452**
**US-A- 3 631 211**
**US-A- 4 251 675**
**US-A- 4 365 103**
**US-A- 5 136 114**

**JOURNAL OF ORGANIC CHEMISTRY. vol. 26, no. 5, Mai 1961, EASTON US Seiten 1398 -1401; A.T. Coscia et al.: "The synthesis of 2,2-ditolylpropane from alpha,p-dimethylstyrene"**

**JOURNAL OF ORGANIC CHEMISTRY. vol. 41, no. 10, 14 Mai 1976, EASTON US Seiten 1698 - 1701; R.M. Roberts et al.: "Friedel-Crafts alkylations with vinyl halides. 1. Vinyl cations and spirobiindans"**

(73) Patentinhaber: **BAYER AG**

**D-51368 Leverkusen (DE)**

(72) Erfinder: **Arlt, Dieter, Prof. Dr.**
**Rybniker Strasse 2**
**D-5000 Köln 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 399 284 B1

Rank Xerox (UK) Business Services
(3.10/3.09/3.3.3)

## Beschreibung

Die Erfindung betrifft ein neues Verfahren zur Herstellung von geminalen Diarylalkanen, und neue Aralkylverbindungen.

1,1-Diarylalkane sind leicht durch säurekatalysierte Alkylierung von aromatischen Verbindungen mit Aldehyden oder Styrolen erhältlich (siehe z.B. J. Org. Chem. 26 (1961) S. 1398). Die Herstellung von geminalen Diarylalkanen mit nicht endständigen geminalen Arylgruppen, die im Prinzip durch Kondensation von aliphatischen Ketonen oder α-Methylstyrolen mit aromatischen Verbindungen erfolgen könnte, ist jedoch auf die Verwendung aktivierter aromatischer Verbindungen wie Phenole und Aniline beschränkt. Toluol z.B. läßt sich nicht mit Aceton oder Methylstyrolen zu den entsprechenden 2,2-Diarylpropanen kondensieren (siehe J. Org. Chem. loc. cit.).

Die Herstellung geminaler Diarylalkane mit nicht endständigen Arylgruppen, z.B. des 2,2-Ditolylpropans, bereitet erhebliche Schwierigkeiten und erfordert für jede aromatische Verbindung jeweils spezielle Alkylierungsmittel und Friedel-Crafts-Katalysatoren (siehe J. Org. Chem. loc. cit. und J. Org. Chem. 41 (1976) S. 1698 ff., insbesondere S. 1701).

Da geminale Diarylalkane, insbesondere auch solche mit nicht endständigen Arylgruppen, ein erhebliches technisches Interesse als Ausgangsverbindungen u.a. für die Herstellung von Kunststoffen (z.B. Polyimiden) besitzen, bestand daher die Aufgabe, ein wirtschaftliches Verfahren aufzufinden, nach dem geminale Diarylalkane aus den verschiedensten aromatischen Verbindungen hergestellt werden könnnen.

Überraschenderweise wurde gefunden, daß bestimmte 1,1,1,3-Tetrachlorpropane oder die aus diesen durch HCl-Abspaltung erhältlichen isomeren 1,1,1-Trichlor-2- oder 1,1,3-Trichlor-1-propene ideale aliphatische Kondensationspartner für aromatische Verbindungen darstellen, weil sie je Mol mit 2 Mol der verschiedensten aromatischen Verbindungen, aktivierten oder nicht aktivierten, in Gegenwart üblicher Friedel-Crafts-Kataylsatoren unter Abspaltung von Vinylidenchlorid zu geminalen Diarylalkanen reagieren. Überraschenderweise bleibt die Friedel-Crafts-Reaktion der Tetrachlorpropane (Trichlorpropene) nicht auf der Stufe der Aralkylverbindungen stehen, sondern es reagieren diese Aralkylverbindungen mit weiterer aromatischer Verbindung unter Abspaltung von Vinylidenchlorid zu den gewünschten geminalen Diarylalkanen.

Diese Umsetzung der erfindungsgemäß zu verwendenden Tetrachlorpropane bzw. Trichlorpropene mit aromatischen Verbindungen zu Diarylalkanen weist gegenüber den bekannten Verfahren den Vorteil auf, daß sie breit, d.h., auf die verschiedensten aromatischen Verbindungen anwendbar ist und die Herstellung von geminalen Diarylalkanen in technischen Mengen ermöglicht, die bislang überhaupt nicht oder nur in Labormengen zugänglich waren, daß sie nur übliche Friedel-Crafts-Katalysatoren erfordert und daß die als Alkylierungsmittel zu verwendenden Halogenalkane bzw. Halogenalkene, durch Addition von $CCl_4$ an petrochemische Grundchemikalien nämlich α-Olefine wie Propen, i-Buten, 2-Methyl-1-buten,2-Methyl-1-penten, 4-Methyl-1-penten, n-Octen, n-Dodecen, n-Heptadecen und n-Octadecen erhältlich und deshalb leicht zugänglich sind. $CCl_4$ fällt, seit organische chlorhaltige Rückstände üblicherweise durch Chlorolyse aufgearbeitet werden, als Chlorolyse-Produkt in reichlicher Menge an und es sind deshalb neue Einsatzgebiete für $CCl_4$ gesucht.

Das erfindungsgemäße Verfahren zeigt einen sinnvollen Weg, um dieses aus Abfallprodukten in großer Menge herstellbare $CCl_4$ einerseits in die gewünschten geminalen Diarylalkane andererseits aber auch in das als Koppelprodukt anfallende Vinylidenchlorid umzuwandeln; Vinylidenchlorid ist ein für die Herstellung wertvoller Copolymerisate (z.B. Modacrylfasern, Saran-Polymere) gefragtes Monomer.

Das heißt, im Zuge der neu gefundenen Umsetzung findet durch die Verwendung der aus $CCl_4$ und Olefinen hergestellten Halogenalkane bzw. Halogenalkene und deren Umsetzung mit aromatischen Verbindungen zu Diarylalkanen und Vinylidenchlorid eine Umwandlung des bei der Rückstandschlorolyse anfallenden $CCl_4$ (d.h., einer $C_1$-Verbindung mit begrenzter Verwendbarkeit) in eine $C_2$-Verbindung mit wesentlich breiterer Verwendbarkeit (Vinylidenchlorid) statt.

Die Erfindung betrifft daher ein Verfahren zur Herstellung von monomeren oder oligomeren geminalen Diarylalkanen der Formel

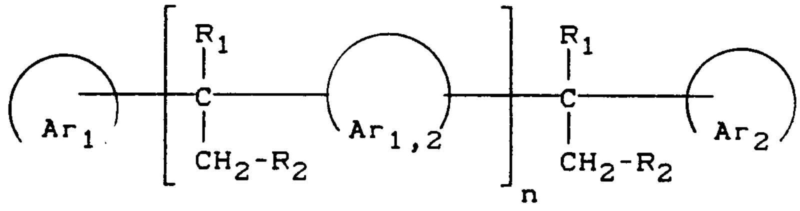 (I),

in der

n eine ganze Zahl von 1 bis 5 oder, vorzugsweise, Null ist,

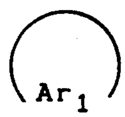 und 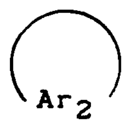

unabhängig voneinander für einen gegebenenfalls substituierten Arylrest stehen und

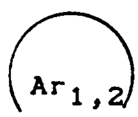

entweder einen

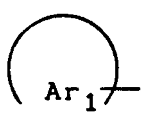 oder 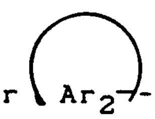-Rest

bedeutet,

$R_1$ für Wasserstoff oder einen $C_1$-$C_{18}$-Alkylrest steht und

$R_2$ Wasserstoff oder einen gegebenenfalls substituierten $C_1$-$C_{18}$-Alkylrest bedeutet

das dadurch gekennzeichnet ist, daß man aromatische Verbindungen der Formeln

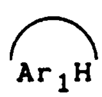 und/oder 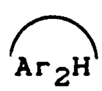 (II),

in der

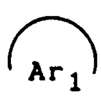 und 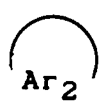

die unter Formel (I) angegebene Bedeutung haben,

mit einer aliphatischen Halogenverbindung der Formeln

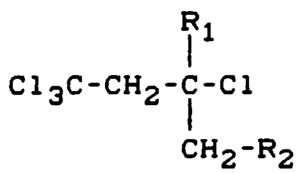 (IIIa),

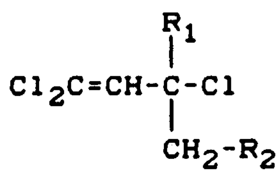 (IIIb),

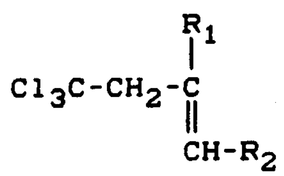 (IIIc),

in denen
$R_1$ und $R_2$ die unter Formel (I) angegebene Bedeutung haben,
in Gegenwart von Friedel-Crafts-Katalysatoren umsetzt.

Die Molverhältnisse, in denen die aromatischen Verbindungen II und die aliphatischen Halogenverbindungen III eingesetzt werden, richten sich nach den gewünschten Endprodukten, ob monomere oder oligomere Diarylalkane gewünscht werden. Für die Herstellung monomerer geminaler Diarylalkane (Verbindungen der Formel (I), in der n Null ist) werden die aromatischen Verbindungen II im Überschuß, vorzugsweise in einer Menge von 10 bis 30 Mol je Mol aliphatischer Halogenverbindung eingesetzt. Für die Herstellung der oligomeren geminalen Diarylalkane (Verbindungen der Formel (I), in der n eine ganze Zahl von 1 bis 5 ist) werden die aromatischen Verbindungen und aliphatischen Halogenalkane je nach gewünschtem Oligomerisierungsgrad im Molverhältnis von 1:1 bis 5:1 angewendet.

Das erfindungsgemäße Verfahren kann sowohl in der Flüssigphase, als auch in der Gasphase durchgeführt werden. Bei der Durchführung des Verfahrens in der Flüssigphase wird bei Temperaturen von -50 bis +200 °C, vorzugsweise -20 bis +150 °C, gearbeitet. Als Friedel-Crafts-Katalysatoren werden die üblicherweise für die Alkylierung von aromatischen Verbindungen verwendeten Friedel-Crafts-Katalysatoren eingesetzt, z.B. $AlCl_3$, $BF_3$, $FeCl_3$, $ZnCl_2$, $TiCl_4$, $ZrCl_4$, $SnCl_4$, $H_3PO_4$, HF, $HBF_4$, saure Erdalkaliphosphate, Tonerden, Zeolithe oder saure Ionenaustauscherharze. Gegebenenfalls kann zur Beschleunigung der Reaktion die Mitverwendung von Cokatalysatoren, wie sie bei der Friedel-Crafts-Alkylierung vielfach verwendet werden, vorteilhaft sein; solch ein Cokatalysator ist z.B. HCl.

Die erfindungsgemäße Flüssigphasenalkylierung kann z.B. wie folgt ausgeführt werden: Die Lösung des Friedel-Crafts-Katalysators in überschüssiger aromatischer Verbindung oder in der mit einem inerten organischen Lösungsmittel, wie Methylenchlorid verdünnten aromatischen Verbindung, wird unter Rühren bei Temperaturen von -10 bis +50 °C mit einem der Halogenalkane der Formel (IIIa-c), einem Gemisch dieser Verbindungen oder einer Lösung der Verbindungen in einem organischen Lösungsmittel versetzt. Das Reaktionsgemisch wird zur Vervollständigung der Umsetzung noch einige Zeit, je nach Ansatzgröße 10 Min. bis 20 Stunden, bei Temperaturen von 0 bis +50 °C gerührt. Der Katalysator wird anschließend durch Zugabe von Wasser desaktiviert. Die organische Phase des Reaktionsgemisches wird abgetrennt und nach dem Trocknen destillativ aufgearbeitet.

Bei Durchführung des erfindungsgemäßen Verfahrens in der Gasphase wird bei Temperaturen von etwa 200 bis 450 °C gearbeitet. Als Katalysatoren werden die üblicherweise für Gasphasenalkylierungen von Aromaten verwendeten Friedel-Crafts-Katalysatoren wie $Al_2O_3 \cdot SiO_2$, $H_3PO_4 \cdot SiO_2$, $BF_3/\gamma Al_2O_3$ und saure Zeolithe angewendet.

Die erfindungsgemäße Gasphasenalkylierung wird wie folgt ausgeführt; Die aromatischen Verbindungen (II) und aliphatischen Halogenverbindungen IIIa-c werden durch Erhitzen in einem geeigneten Apparat, z.B. einem Schlangenrohr- oder Fallfilm-Verdampfer, in den gasförmigen Zustand gebracht und in einen für kontinuierliche Gasphasen-Prozesse geeigneten Reaktor, z.B. einen Rohrbündel-Reaktor, überführt, der den

Alkylierungskatalysator in fester Form enthält. Das den Reaktor verlassende Reaktionsgemisch wird durch Kondensation in flüssige und gasförmige Produkte aufgetrennt.

Die reinen Stoffe werden durch fraktionierte Destillation erhalten, wobei Chlorwasserstoff durch Waschprozesse, z.B. mit Wasser, gegebenenfalls zuvor entfernt werden kann.

Die dem erfindungsgemäßen Verfahren zugrundeliegende Kondensationsreaktion verlauft in zwei Stufen:

Stufe 1:

(III) 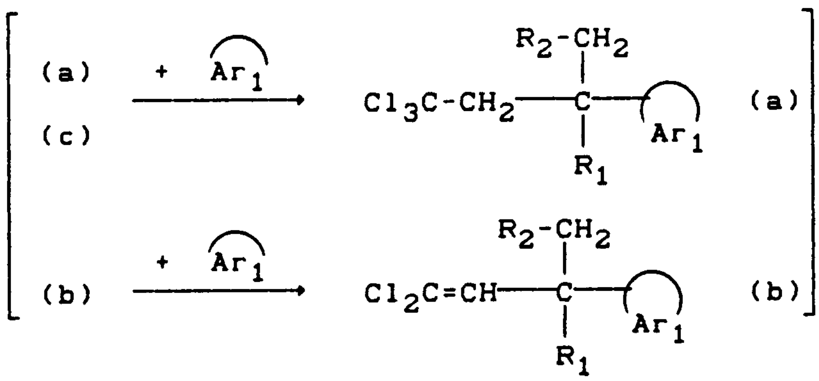 (IV)

Stufe 2:

(IV) 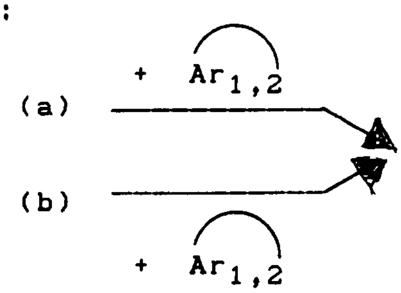 (I)

Das erfindungsgemäße Verfahren kann ein- oder zweistufig durchgeführt werden, je nachdem, ob man die Alkylierungsreaktion auf der Stufe der Aralkylverbindungen 1Va oder b anhalten oder ob man unmittelbar die geminalen Diarylalkane der Formel (1) erhalten möchte. Die zweistufige Arbeitsweise wird bevorzugt für die Herstellung von geminalen Diarylalkanen der Formel (I) angewendet, in der

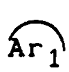 und 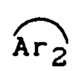

für verschiedene Arylreste stehen.

Bei zweistufiger Arbeitsweise wird die Kondensation der aromatischen Verbindungen in der ersten Reaktionsstufe mit den aliphatischen Halogenverbindungen IIIa-c unter Verwendung schwächer wirksamer Friedel-Crafts-Katalysatoren wie $ZnCl_2$, $ZnJ_2$ oder $SnCl_4$ und/oder bei niedrigeren Temperaturen wie -70° bis +10°C und in kürzeren Reaktionszeiten vorgenommen.

Für die zweite Stufe, die Kondensation der Aralkylverbindungen IVa oder b mit weiterer aromatischer Verbindung II, werden, wie für die einstufige Arbeitsweise, stärkere Friedel-Crafts-Katalysatoren wie $AlCl_3$ und höhere Temperaturen wie +25°C bis +150°C angewendet.

Wie bereits betont, eignet sich das erfindungsgemäße Verfahren für die Herstellung von geminalen Diarylalkanen aus den verschiedensten aromatischen Verbindungen. Als aromatische Verbindungen kommen z.B. Benzol, Toluol, o-, m- und p-Xylol, Diphenylmethan, 2,2-Diphenylpropan, Diphenyl, 4-Methyldiphenyl, Naphthalin, 1- und 2-Methylnaphthalin, 2,3-Dimethyl-naphthalin, Acenaphten, Chlorbenzol, Brombenzol, Fluorbenzol, Phenol, o-, m- und p-Kresol, Anisol, Phenetol, Diphenylether, Thiophenol und Diphenylthioether in Betracht.

Dementsprechend seien als gegebenenfalls substituierte Arylreste

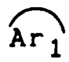 und 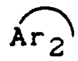

beispielsweise genannt:
gegebenenfalls durch $C_1$-$C_4$-Alkyl wie Methyl, Ethyl, Isopropyl; Hydroxy; $C_1$-$C_4$-Alkoxy wie Methoxy und Ethoxy; Phenoxy; Mercapto; $C_1$-$C_4$-Alkylmercapto wie Methylmercapto; Phenylmercapto; und durch Halogen wie Fluor, Chlor oder Brom substituierte Phenyl-, Biphenyl-, Diphenylmethan-, 2,2-Diphenylpropan- und Naphthyl-Reste, ferner der Acenaphthyl-Rest.

Als $C_1$-$C_{18}$-Alkylreste seien für $R_1$ und $R_2$ genannt: der Methyl-, Ethyl-, Propyl-, Butyl-, Octyl-, Dodecyl-, Heptadecyl- und Octadecyl-Rest; als Substituenten kommen für $R_2$ vor allen Dingen Halogenatome wie Chloratome in Betracht.

Die nach dem erfindungsgemäßen Verfahren erhältlichen geminalen Diarylalkane der Formel (I) sind großenteils neu. Die neuen monomeren Verbindungen sind wertvolle Zwischenprodukte für die Herstellung von Kunststoffen, z.B. von Polyimiden. Die neuen geminalen Diarylalkane, insbesondere die 2,2-Diarylpropane, deren Arylgruppen durch zwei vicinale Methylgruppen substituiert sind, eröffnen die Möglichkeit zur Herstellung neuer bislang nicht herstellbarer Polyimide mit neuen interessanten Eigenschaften. Die vicinalen Methylgruppen der Arylreste werden durch Oxidation in Carboxylgruppen überführt, die so erhaltenen Tetracarbonsäuren mit Diaminen zu Polyimiden umgesetzt.

Die neuen oligomeren Diarylalkane eignen sich als Elektroisolier- und Wärmeüberträgerflüssigkeiten.

Die neuen geminalen Diarylalkane entsprechen der Formel

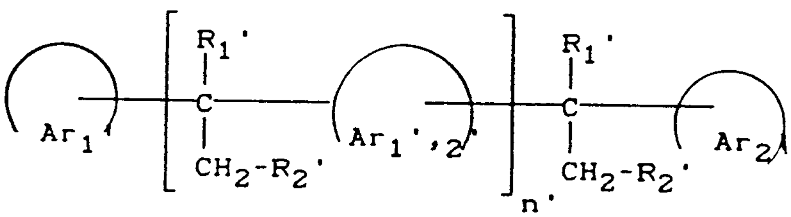 (VI),

in der für den Fall, daß

n' eine ganze Zahl von 1 bis 5 ist,

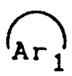 und 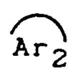,

unabhängig voneinander für einen gegebenenfalls substituierten Arylrest stehen und

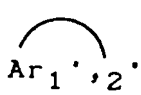

entweder einen

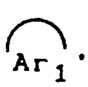- oder 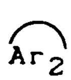-Rest

bedeutet, mit der Maßgabe, daß wenn

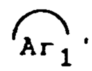

Phenyl oder Hydroxyphenyl ist

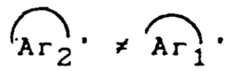

ist,

R1' für Wasserstoff oder einen $C_1$-$C_{18}$-Alkylrest steht und

R2' Wasserstoff oder einen gegebenenfalls substituierten $C_1$-$C_{18}$-Alkylrest bedeutet, und in der für den Fall, daß

n' Null ist,

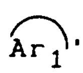 für 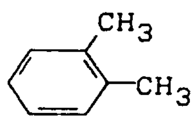 , 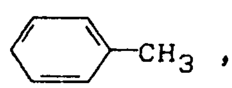 , 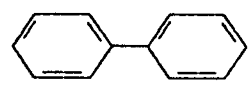 ,

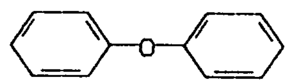 und

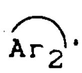 für 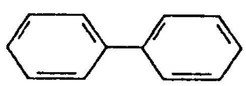 , 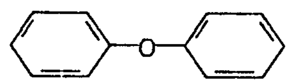 , 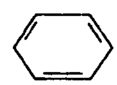

R1' für Methyl und

R2' für Wasserstoff oder Methyl stehen.

Als Vertreter der neuen Diarylalkane der Formel (VI), wenn in dieser n' = O ist, seien beispielsweise genannt: 1-Phenyl-1-xylyl-ethan, 2-Phenyl-2-xylyl-propan, 2,2-Bis-(4-chlorphenyl)-propan, 2,2-Bis-(biphenyl)-propan, 2-Biphenyl-2-xylyl-propan, 1-Biphenyl-1-tolyl-ethan, 2-Biphenyl-2-tolyl-propan, 2-Biphenyl-2-phenyl-propan, 2-Phenyl-2-tolyl-propan, 2,2-Bis-(p-phenoxy-phenyl)propan, 2-Phenyl-2-(p-phenoxy-phenyl)-propan und 2-(p-Phenoxy-phenyl)-2-xylyl-propan.

Die bei der zweistufigen Durchführung des erfindungsgemäßen Verfahrens erhältlichen Aralkylverbindungen der Formeln (IVa und b) sind ebenfalls neu; es wurde gefunden, daß diese nicht nur Zwischenprodukte für die Herstellung insbesondere unsymmetrischer geminaler Diarylalkane der Formel (I) sind, sondern daß sie auch interessante Zwischenprodukte für die Herstellung von Pflanzenschutzmitteln sind und z.B. einen neuen günstigereren Weg zur Herstellung bekannter Wirkstoffe eröffnen.

Die Erfindung betrifft daher auch die Aralkylverbindungen der Formeln (IVa und b), in denen

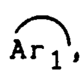

$R_1$ und $R_2$ die unter Formel (I) angegebene Bedeutung haben.

Als Vertreter dieser neuen Aralkylverbindungen seien beispielsweise genannt:

1,1,1-Trichlor-3-methyl-3-phenyl-butan
1,1,1-Trichlor-3-methyl-3-tolyl-butan
1,1,1-Trichlor-3-methyl-3-xylyl-butan
1,1,1-Trichlor-3-methyl-3-chlorphenyl-butan
1,1,1-Trichlor-3-methyl-3-bromphenyl-butan
1,1,1-Trichlor-3-methyl-3-fluorphenyl-butan

1,1,1-Trichlor-3-methyl-3-ethoxyphenyl-butan
1,1,1-Trichlor-3-methyl-3-hydroxyphenyl-butan
1,1,1-Trichlor-3-methyl-3-mercaptophenyl-butan
1,1,1-Trichlor-3-methyl-3-methylmercapto-phenyl-butan
1,1,1-Trichlor-3-methyl-3-tolyl-butan
1,1,1-Trichlor-3-methyl-3-xylyl-pentan
1,1,1-Trichlor-3-methyl-3-chlorphenyl-pentan
1,1,1-Trichlor-3-methyl-3-fluorphenyl-pentan
1,1,1-Trichlor-3-methyl-3-ethoxyphenyl-pentan
1,1,1-Trichlor-3,4-dimethyl-3-tolyl-pentan
1,1,1-Trichlor-3,4-dimethyl-3-xylyl-pentan
1,1,1-Trichlor-3,4-dimethyl-3-fluorphenyl-pentan
1,1,1-Trichlor-3,4-dimethyl-3-ethoxyphenyl-pentan
1,1,1-Trichlor-3-methyl-3-biphenyl-butan
1,1,1-Trichlor-3-methyl-3-biphenyl-pentan
1,1,1-Trichlor-3-methyl-3-naphthyl-butan
1,1,1-Trichlor-3-methyl-3-dimethylnaphthyl-butan
1,1,1-Trichlor-3-methyl-3-acenaphthyl-butan
1,1,1-Trichlor-3-methyl-3-phenoxyphenyl-butan
1,1-Dichlor-3-phenyl-buten
1,1-Dichlor-3-tolyl-buten
1,1-Dichlor-3-hydroxphenyl-buten
1,1-Dichlor-3-methoxyphenyl-buten
1,1-Dichlor-3-fluorphenyl-buten
1,1-Dichlor-3-cumyl-buten
1,1-Dichlor-3-biphenyl-buten
1,1-Dichlor-3-phenyl-penten
1,1-Dichlor-3-tolyl-penten
1,1-Dichlor-3-methoxyphenyl-penten
1,1-Dichlor-3-ethoxyphenyl-penten
1,1-Dichlor-3-hydroxyphenyl-penten
1,1-Dichlor-3-phenyl-hepten
1,1-Dichlor-3-tolyl-undecen
1,1-Dichlor-3-phenyl-heptadecen
1,1-Dichlor-3-phenyl-nonadecen
1,1-Dichlor-3-methyl-3-phenyl-buten
1,1-Dichlor-3-methyl-3-tolyl-buten
1,1-Dichlor-3-methyl-3-chlorphenyl-buten
1,1-Dichlor-3-methyl-3-xylyl-buten
1,1-Dichlor-3-methyl-3-ethoxyphenyl-buten
1,1-Dichlor-3-methyl-3-hydroxyphenyl-buten
1,1-Dichlor-3-methyl-3-tolyl-penten
1,1-Dichlor-3-methyl-3-ethoxyphenyl-penten
1,1-Dichlor-3-methyl-3-xylyl-penten
1,1-Dichlor-3,4-dimethyl-3-phenyl-penten
1,1-Dichlor-3,4-dimethyl-3-tolyl-penten
1,1-Dichlor-3,4-dimethyl-3-xylyl-penten
1,1-Dichlor-3,4-dimethyl-3-fluorphenyl-penten
1,1-Dichlor-3,4-dimethyl-3-ethoxyphenyl-penten

Die Bedeutung der Verbindungen IVa und b als Zwischenprodukte für die Herstellung von Pflanzenschutzmitteln sei am Beispiel der Herstellung von Insektiziden des Typs MTI 800 (siehe DE-OS 33 17 907) erläutert. Für die Herstellung dieser aromatischen Alkanderivate wird ein Vielstufenverfahren beschrieben, das komplizierte Reaktionen und den Einsatz teurer, in technischem Maßstab nur schwierig handhabbarer Reagentien wie Grignard-Verbindungen und $LiAlH_4$ erfordert (siehe DE-OS 33 17 908, das Reaktionsschema auf S. 62/63). Mit Hilfe der erfindungsgemäßen Aralkylverbindungen der Formeln IVa und b läßt sich nicht nur die Zahl der Reaktionsstufen wesentlich verringern, sondern die einzelnen Reaktionen sind auch ohne Schwierigkeiten in technischem Maßstab durchführbar, weil sie nur einfache, in der chemischen Technik üblicherweise verwendete Reagentien erfordern.

Das heißt, mit Hilfe der erfindungsgemäßen Aralkylverbindungen der Formel IVa und b lassen sich die in der DE-OS 33 17 908 beschriebenen Wirkstoffe auf vereinfachtem, wesentlich wirtschaftlicherem Weg herstellen.

Beispiel 1

Die Lösung von 2,5 g (0,019 Mol) $AlCl_3$ in 10 ml Dichlormethan wird bei Temperaturen von 0 bis 5°C innerhalb von 15 Min. unter Rühren und Kühlen mit einer Lösung von 25 g (0,12 Mol) 1,1,1,3-Tetrachlor-3-methylbutan in 250 g (3,2 Mol) Benzol versetzt. Das Reaktionsgemisch wird 100 Min. bei 5°C gerührt. Nach Zugabe von 50 ml Wasser wird die organische Phase abgetrennt, getrocknet und fraktioniert destilliert.

Es werden 268 g Rohprodukt erhalten, das gemäß GC-Analyse 4,1 % Vinylidenchlorid (= 11 g = 94,8 % der Theorie) enthält. Durch fraktionierte Destillation des Rohproduktes werden 20,4 g (= 87 % der Theorie) 2,2-Diphenyl-propan (Kp.: 89°C/0,1 mbar) erhalten.

Beispiel 2

Die Lösung von 20 g (0,15 Mol) $AlCl_3$ in 2500 g (27 Mol) getrocknetem Toluol wird bei 0°C innerhalb von 10 Min. unter Rühren und Kühlen mit 500 g (2,38 Mol) 1,1,1,3-Tetrachlor-3-methyl-butan versetzt. Das Reaktionsgemisch wird 60 Min. bei 0°C gerührt und nach Zugabe von 200 ml Wasser wird die organische Phase abgetrennt, mit Natriumsulfat getrocknet und durch fraktionierte Destillation aufgearbeitet.

Es werden 145 g (= 72 % der Theorie) Vinylidenchlorid, 90 g nicht umgesetztes 1,1,1,3-Tetrachlor-3-methyl-butan und 410 g (= 94 % der Theorie) 2,2-Di-tolyl-propan (Isomerengemisch; Kp.: 106-115°C/0,1 mbar) erhalten. Gehalt des Isomerengemisches an p,p'-Isomer: 65 %.

Beispiel 3

Die Lösung von 2,5 g (0,015 Mol) wasserfreiem $FeCl_3$ in 1400 g (15,2 Mol) wasserfreiem Toluol wird bei Raumtemperatur unter Rühren mit 100 g (0,48 Mol) 1,1,1,3-Tetrachlor-3-methyl-butan versetzt. Die Reaktionsmischung wird 60 Min. bei 50 bis 60°C und anschließend 180 Min. bei Rückflußtemperatur gerührt. Nach Zugabe von Wasser wird die organische Phase abgetrennt, getrocknet und durch Destillation aufgearbeitet.

Es werden 80,8 g (= 73,5 % der Theorie) 1,1-Dichlor-3-tolyl-3-methyl-buten-(1) erhalten (Kp.: 85-89°C/0,2 mbar).

Beispiel 4

Die Lösung von 50 g (0,38 Mol) $AlCl_3$ in 5000 g (44,6 Mol) trockenem Chlorbenzol wird bei Temperaturen von 0 bis 10°C unter Rühren und Kühlen innerhalb von 30 Min. mit 500 g (2,38 Mol) 1,1,1,3-Tetrachlor-3-methyl-butan versetzt. Die Reaktionsmischung wird weitere 90 Min. bei 10°C gerührt. Nach Zugabe von 500 ml Wasser wird die organische Phase abgetrennt, mit Sodalösung neutral gewaschen und mit Natriumsulfat getrocknet.

Bei der fraktionierten Destillation werden erhalten; 4750 g Vorlauf; er enthält gemäß GC-Analyse 4,5 % (= 213 g = 92,6 % der Theorie) Vinylidenchlorid; 532 g (= 84,3 % der Theorie) 2,2-Bis-(chlorphenyl)-propan (überwiegend p,p'-Isomer) (Kp.: 160-165°C/0,8 mbar) und 84 g (= 14 % der Theorie) 1,1-Dichlor-3-chlorphenyl-3-methylbuten (überwiegend p,p'-Isomer) (Kp.: 115-118°C/0,3 mbar).

Beispiel 5

Die Lösung von 2 g (0,015 Mol) $AlCl_3$, 462 g (3,0 Mol) Diphenyl und 106 g (1,0 Mol) o-Xylol in 450 ml Methylenchlorid wird unter Rühren mit 30 g (0,14 Mol) 1,1,1,3-Tetrachlor-3-methyl-butan versetzt. Das Reaktionsgemisch wird insgesamt 20 Stunden bei 10°C gerührt, wobei nach 5 und 7 Stunden jeweils noch 2 g $AlCl_3$ nachträglich zugesetzt wurden. Nach Zugabe von 50 ml Wasser wird die organische Phase abgetrennt und nach dem Trocknen durch Destillation aufgearbeitet.

Es werden nicht umgesetztes o-Xylol und Diphenyl zurückgewonnen. Es werden 13,8 g (= 32 % der Theorie) einer überwiegend aus 2-Biphenyl-2-xylyl-propan bestehenden Fraktion vom Siedebereich 210-225°C/0,2 mbar und 15 g (= 30 % der Theorie) einer im wesentlichen aus 2,2-Bis(biphenyl)-propan bestehenden Fraktion vom Siedebereich 225-250°C/0,2 mbar erhalten. Fp. des durch Umkristallisieren aus

Hexan gereinigten 2,2-Bis-(biphenyl)-propans: 138-139 °C.

Beispiel 6

Die Lösung von 2,8 g $AlCl_3$ (0,02 Mol) in 300 ml (3,0 Mol) trockenem Benzol wird bei 0 °C innerhalb von 30 Min. unter Rühren und Kühlen mit 30 g (0,14 Mol) 1,1,3-Trichlor-buten-1 versetzt. Das Reaktionsgemisch wird weitere 90 Min. bei 0 bis 5 °C gerührt. Nach Zugabe von 50 ml Wasser wird die organische Phase abgetrennt und durch fraktionierte Destillation aufgearbeitet.

Es werden 27 g (= 71 % der Theorie) 1,1-Dichlor-3-phenyl-buten-1 (Kp.: 73-74 °C/0,15 mbar) und 2,6 g (= 7,6 % der Theorie) 1,1-Bisphenyl-ethan (Kp.: 87-89 °C/0,1 mbar) erhalten.

Beispiel 7

Es wurde wie in Beispiel 6 beschrieben gearbeitet mit dem Unterschied, daß die Reaktionstemperatur 25 °C und die Reaktionszeit 18 Stunden betrug. Der unter Normaldruck abdestillierte Vorlauf (210 g) enthält gemäß GC-Analyse 5,9 % (= 13,4 g = 91 % der Theorie) Vinylidenchlorid. Bei der nachfolgenden Vakuumdestillation werden 18,2 g (= 53 % der Theorie) 1,1-Bisphenylethan (Kp.: 88-90 °C/0,1 mbar) erhalten.

Beispiel 8

Die Lösung von 500 g Diphenylether in 300 ml Dichlormethan wird unter Rühren und Kühlen zunächst mit 5 g $AlCl_3$ und danach mit 30 g 1,1,1,3-Tetrachlor-3-methylbutan versetzt. Nach 5 stündigem Rühren werden erneut 5 g $AlCl_3$ hinzugefügt und das Reaktionsgemisch auf 22 °C erwärmt. Nach weiteren 10 Stunden Reaktionszeit werden 200 ml Wasser zugetropft und die Phasen getrennt. Die mit Natriumsulfat getrocknete organische Phase wird destilliert.
Es werden 33,3 g (= 61 % der Theorie) 2,2-Bis-(4-phenoxyphenyl)-propan (Kp.: 225-230 °C/0,6 mbar) erhalten.

Beispiel 9

Die Lösung von 154 g Biphenyl in 312 g Benzol wird bei 0 °C mit 1 g $AlCl_3$ und 30 g 1,1,1,3-Tetrachlor-3-methylbutan versetzt. Nach 5 stündigem Rühren werden weitere 2 g $AlCl_3$ zugegeben und das Reaktionsgemisch weitere 10 Stunden bei 20-25 °C gerührt. Nach Zugabe von 50 ml Wasser werden die Phasen getrennt. Die organische Phase wird durch Destillation aufgearbeitet.
Es werden neben 2,2-Diphenylpropan und 2,2-Bis-(biphenyl)-propan 13,5 g 2-Biphenyl-2-phenyl-propan (Kp.: 190-195 °C/0,05 mbar) erhalten.

Beispiel 10

Die Mischung von 2400 ml Benzol, 600 ml o-Xylol und 12 g $AlCl_3$ wird langsam unter Rühren und Kühlen bei 0 °C mit 300 g 1,1,1,3-Tetrachlor-3-methyl-butan versetzt. Nach 5 stündigem Rühren wird die Reaktion durch Zugabe von 250 ml Wasser beendet. Die organische Phase wird durch Destillation aufgearbeitet. Zunächst werden unter Normaldruck Vinylidenchlorid, Benzol und o-Xylol abdestilliert; anschließend werden durch Vakuumdestillation 290 g unumgesetztes 1,1,1,3-Tetrachlor-3-methyl-butan, 33 g 2-Phenyl-2-o-xylyl-propan (Siedebereich: 130 °C-134 °C/0,4 mbar) und 61 g 2,2-Bis-(o-xylyl)-propan (Kp.: 158 °C-160 °C/0,2 mbar) erhalten.

Beispiel 11

In 2700 ml wasserfreiem o-Xylol werden bei 0 °C 25 g $AlCl_3$ unter Rühren gelöst. Dann werden innerhalb von 15 Minuten 270 g 1,1,1,3-Tetrachlor-3-methyl-butan unter Rühren zugetropft; das Reaktionsgemisch wird anschließend zunächst eine Stunde bei 0 °C-10 °C, dann eine weitere Stunde bei 20 °C gerührt. Durch Zugabe von 1000 ml Wasser wird die Reaktion beendet. Die abgetrennte organische Phase wird durch Destillation aufgearbeitet. Es werden neben Vinylidenchlorid und Xylol 305 g (= 94 % der Theorie) 2,2-Bis-(o-xylyl)-propan (Kp.: 152 °C-155 °C/0,15 mbar) erhalten.

Beispiel 12

a) Die Suspension von 40 g $AlCl_3$ in 100 ml Dichlormethan wird unter Rühren und Kühlen bei -50 °C innerhalb von 20 Min. mit der Lösung von 235 g (= 1,12 Mol) 1,1,1,3-Tetrachlor-3-methyl-butan in 800 g (8,7 Mol) Toluol versetzt. Nach 40-minütigem Rühren bei -50 °C wird das Reaktionsgemisch mit Wasser versetzt und die organische Phase abgetrennt. Nach dem Auswaschen und Trocknen der organischen Phase wird diese im Rotationsverdampfer vom Lösungsmittel und überschüssigem Toluol befreit. Es werden 299 g Rohprodukt erhalten, das gemäß GC-Analyse 82 % (= 245 g = 82,5 % der Theorie) 1,1,1-Trichlor-3-methyl-3-p-tolyl-butan enthält. Durch Destillation des Rohproduktes wird die reine Verbindung (Kp.: 89 °C/0,04 mbar) erhalten.

b) Verwendung des 1,1,1-Trichlor-3-methyl-3-p-tolylbutan zur Herstellung von 1-[4-Fluor-3-phenoxyphenyl]-4-methyl-4-p-tolyl-pentan.

α) Das Rohprodukt aus Stufe a) wird mit einer Lösung von 300 g Natriumhydroxid in 240 ml Wasser unter Zugabe von 3 g Tetrabutylammoniumbromid durch kräftiges Rühren vermischt und 20 Stunden unter Rühren auf 100 °C erwärmt. Das Reaktionsgemisch wird mit Dichlormethan extrahiert; die vereinigten Extrakte werden mit verdünnter Salzsäure gewaschen und nach dem Trocknen durch Destillation aufgearbeitet. Es werden 140 g (= 78,8 % der Theorie) 1-Chlor-3-methyl-3-p-tolyl-butin(Kp.: 114-115 °C/20 mbar) erhalten.

β) Die Lösung von 96,5 g (0,5 Mol) 1-Chlor-3-methyl-3-p-tolyl-butin in 500 ml absolutem Methanol wird nach Zugabe von 90 g aktiviertem Zinkstaub 72 Stunden unter Rühren auf Rückflußtemperatur erhitzt. Anschließend wird das Reaktionsgemisch im Rotationsverdampfer eingeengt; der Rückstand wird mit verdünnter Salzsäure und Dichlormethan aufgenommen und die organische Phase abgetrennt. Die organische Phase wird durch Destillation aufgearbeitet; es werden 66 g (= 83 % der Theorie) 3-Methyl-3-p-tolyl-butin (Kp.: 90-92 °C/22 mbar) erhalten.

γ) Die Lösung von 32 g (0,2 Mol) 3-Methyl-3-p-tolyl-butin in 150 ml absolutem Tetrahydrofuran wird unter Rühren bei -70 °C mit der Lösung von 0,2 Mol Butyllithium in Hexan und anschließend bei -30 °C mit der Lösung von 43,2 g (0,2 Mol) 4-Fluor-3-phenoxy-benzaldehyd in 100 ml Tetrahydrofuran versetzt. Die Reaktionsmischung wird auf Raumtemperatur erwärmt und 12 Stunden bei dieser Temperatur gerührt. Dann wird das Lösungsmittel im Vakuum entfernt und der Rückstand mit Wasser und Dichlormethan aufgenommen. Die organische Phase wird abgetrennt und durch Destillation aufgearbeitet. Es werden 72 g Rohprodukt erhalten, das gemäß GC-MS-Analyse im wesentlichen aus 1-[4-Fluor-3-phenoxy-phenyl]-3-methyl-3-p-tolyl-pent-2-in-1-ol besteht (Nebenprodukt: [4-Fluor-3-phenoxy-phenyl]-[3-methyl-3-p-tolyl-butin-1-yl]-keton).

δ) Das Rohprodukt wird in 2000 ml Methanol gelöst und die Lösung nach Zusatz von 7 g Pd-C (5 %) bei 80 °C und einem Wasserstoff-Druck von 40 bar in einem Rührautoklaven hydriert. Nach dem Abtrennen des Pd-Katalysators und Einengen der Reaktionslösung im Rotationsverdampfer werden 69 g eines Rohproduktes erhalten, das gemäß GC-MS-Analyse 93 % (= 64,2 g = 88,6 % der Theorie) 1-[4-Fluor-3-phenoxy-phenyl]-4-methyl-4-p-tolyl-pentan ($n_D^{20}$ : 1,5629) enthält.

Beispiel 13

a) Die Suspension von 66,5 g (0,5 Mol) $AlCl_3$ in 100 ml Dichlormethan wird bei 0 °C mit der Mischung von 61 g (0,5 Mol) Phenetol und 105 g (0,5 Mol) 1,1,1,3-Tetrachlor-3-methyl-butan langsam unter Rühren versetzt. Nach 4-stündigem Rühren wird die Reaktionsmischung unter Eiskühlung mit 1000 ml Wasser versetzt. Das Gemisch wird mehrfach mit Dichlormethan extrahiert und die vereinigten Extrakte nach dem Trocknen durch Destillation aufgearbeitet. Es werden neben nicht umgesetztem Ausgangsmaterial 30 g (= 20 % der Theorie) 1,1,1-Trichlor-3-p-ethoxyphenyl-3-methyl-butan (Kp.: 145-150 °C/0,1 mbar) erhalten.

b) Verwendung des 1,1,1-Trichlor-3-p-ethoxyphenyl-3-methyl-butan zur Herstellung von 1-[4-Fluor-3-phenoxy-phenyl]-4-[p-ethoxyphenyl]-4-methylpentan.

α) Die Mischung aus dem in Stufe a) erhaltenen 1,1,1-Trichlor-3-p-ethoxyphenyl-3-methylbutan, 25 g Kalium-tert.-butylat und 200 ml Dimethylformamid wird unter Rühren 12 Stunden auf 85 °C erwärmt. Anschließend werden tert. Butanol und Dimethylformamid durch Vakuumdestillation aus dem Reaktionsgemisch entfernt. Der Rückstand wird in Wasser und Dichlormethan aufgenommen und die organische Phase abgetrennt. Durch Vakuumdestillation der organischen Phase werden 20 g (= 89 % der Theorie) 1-Chlor-3-ethoxyphenyl-3-methyl-butin (Kp.: 98-100 °C/0,07 mbar) erhalten.

β) Die Suspension von 20 g aktiviertem Zinkpulver in 120 ml Methanol wird mit den 20 g 1-Chlor-3-ethoxyphenyl-3-methyl-butin versetzt. Die Mischung wird unter Rühren 24 Stunden auf Rückflußtemperatur erhitzt. Anschließend wird das Methanol abdestilliert, der Rückstand in verdünnter Salzsäure und Dichlormethan aufgenommen und die organische Phase abgetrennt. Die Destillation der organischen Phase liefert 14 g (= 83 % der Theorie) 3-[p-Ethoxyphenyl]-3-methyl-butin-1 (Kp.: 71-73 °C/0,01 mbar).

γ) 1,9 g (0,01 Mol) 3-[p-Ethoxyphenyl]-3-methylbutin-1 in 10 ml absolutem Tetrahydrofuran wird bei -30 °C unter Rühren mit 0,01 Mol einer Lösung von Butyllithium in Hexan versetzt. Nach 3-stündigem Rühren wird das Reaktionsgemisch mit der Lösung von 2,2 g (0,01 Mol) 4-Fluor-3-phenoxy-benzaldehyd in 10 ml Tetrahydrofuran versetzt. Das Reaktionsgemisch wird auf Raumtemperatur erwärmt und nach 5-stündigem Rühren bei dieser Temperatur mit Wasser und Dichlormethan (je 100 ml) versetzt. Die organische Phase wird abgetrennt und im Vakuum eingeengt. Es werden 3,6 g Rohprodukt erhalten, das gemäß GC-MS-Analyse 85 % 1-[4-Fluor-3-phenoxy-phenyl]-4-[p-ethoxyphenyl]-4-methyl-pent-1-in-ol-1 (neben 7 % des entsprechenden Ketons) enthält. Das Rohprodukt wird unter den im Beispiel 8 δ) beschriebenen Bedingungen katalytisch hydriert. Es werden 3 g 1-[4-Fluor3-phenoxy-phenyl]-4-[p-ethoxyphenyl]-4-methylpentan (gemäß GC-MS-Analyse 89 %ig = 2,7 g = 69 % der Theorie) erhalten. Die Verbindung wird durch Chromatographie an Kieselgel rein erhalten ($n_D^{20}$ : 1,5580).

## Patentansprüche

**1.** Verfahren zur Herstellung von monomeren oder oligomeren geminalen Diarylalkanen der Formel

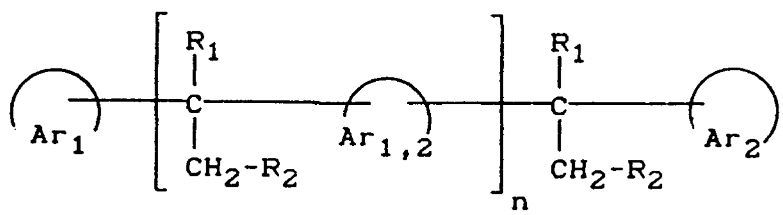

(I),

in der

n eine ganze Zahl von 1 bis 5 oder Null ist,

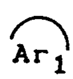 und 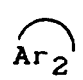

unabhängig voneinander für einen gegebenenfalls substituierten Arylrest stehen und

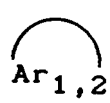

entweder einen

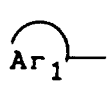 oder 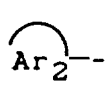-Rest

bedeutet,

$R_1$ für Wasserstoff oder einen $C_1$-$C_{18}$-Alkylrest steht und

$R_2$ Wasserstoff oder einen gegebenenfalls substituierten $C_1$-$C_{18}$-Alkylrest bedeutet,

dadurch gekennzeichnet, daß man aromatische Verbindungen der Formeln

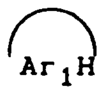 und/oder 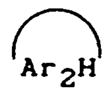 (II),

in der

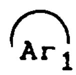 und 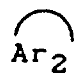

die unter Formel (I) angegebene Bedeutung haben,
mit einer aliphatischen Halogenverbindung der Formeln

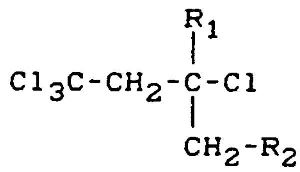 (IIIa),

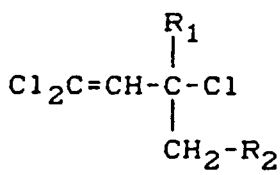 (IIIb),

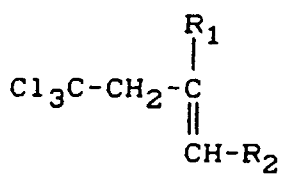 (IIIc),

in denen
$R_1$ und $R_2$ die unter Formel (I) angegebene Bedeutung haben,
in Gegenwart von Friedel-Crafts-Katalysatoren umsetzt.

**2.** Verfahren nach Anspruch 1 dadurch gekennzeichnet, daß man die Umsetzung in der Flüssigphase vornimmt.

**3.** Verfahren nach Anspruch 2 dadurch gekennzeichnet, daß man die Umsetzung bei Temperaturen von -50 bis +200 °C vornimmt.

**4.** Verfahren nach Anspruch 2, dadurch gekennzeichnet, daß man die Umsetzung in einem inerten organischen Lösungsmittel oder in einem Überschuß an flüssiger aromatischer Verbindung vornimmt.

**5.** Verfahren nach einem der Ansprüche 1 bis 4, dadurch gekennzeichnet, daß man zur Herstellung von monomeren Diarylalkanen (Diarylalkanen der Formel (I), in der n Null ist) aromatische Verbindungen der Formeln (II) und aliphatische Halogenverbindungen der Formeln (III) im Molverhältnis 10 bis 30:1 einsetzt.

**6.** Aralkylverbindungen der Formeln

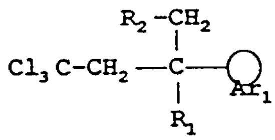 (a)

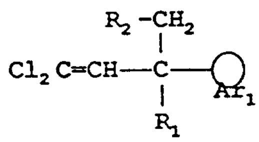 (b)

in denen

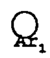

für einen Arylrest, der durch $6_1$-$C_4$-Alkyl, Hydroxy, $C_1$-$C_4$-Alkoxy, Phenoxy, Mercapto, $C_1$-$C_4$-Alkylmercapto, Phenylmercapto, Halogen substituiert sein kann, oder für Acenaphthyl,
$R_1$ für Wasserstoff oder einen $C_1$-$C_{18}$-Alkylrest
$R_2$ für Wasserstoff oder einen gegebenenfalls durch Halogen substituierten $C_1$-$C_{18}$-Alkylrest stehen.

**7.** Aralkylverbindungen der Formeln

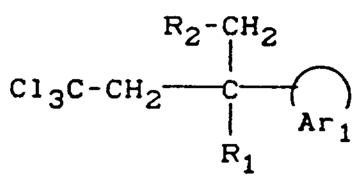 (a)

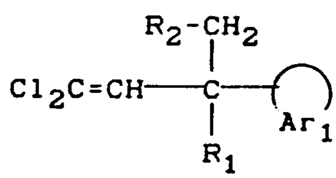 (b)

in denen

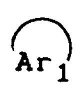

für einen gegebenenfalls substituierten Arylrest,
$R_1$ für Wasserstoff oder einen $C_1$-$C_{18}$-Alkylrest und
$R_2$ für Wasserstoff oder einen gegebenenfalls substituierten $C_1$-$C_{18}$-Alkylrest stehen.

**8.** Aralkylverbindungen gemäß Anspruch 7 dadurch gekennzeichnet, daß
$R_1$ für Wasserstoff, Methyl, Ethyl, n-Propyl oder i-Propyl und
$R_2$ für Wasserstoff oder Methyl stehen.

## Claims

1. Process for the preparation of monomeric or oligomeric geminal diarylalkanes of the formula

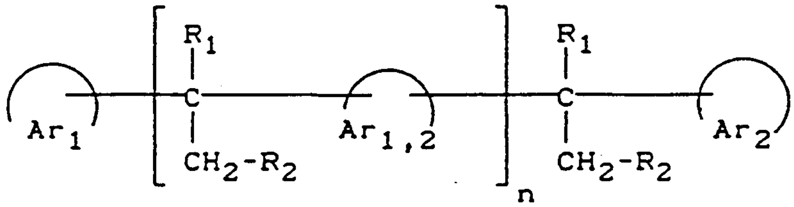

(I)

in which

n is an integer from 1 to 5 or zero,

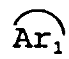 and 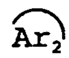

independently of one another represent an optionally substituted aryl radical and

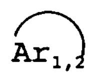

denotes either an

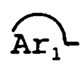 or 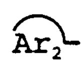

radical,

$R_1$ represents hydrogen or a $C_1$-$C_{18}$-alkyl radical and

$R_2$ represents hydrogen or an optionally substituted $C_1$-$C_{18}$-alkyl radical,

characterized in that aromatic compounds of the formulae

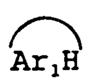 and/or 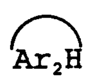 (II)

in which

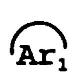 and 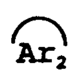

have the meaning indicated under formula (I),

are reacted with an aliphatic halogen compound of the formulae

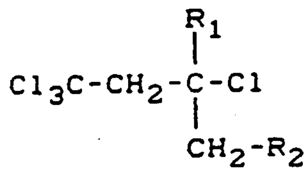 (IIIa)

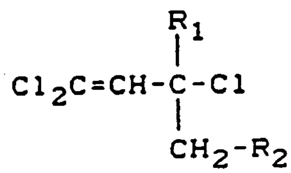 (IIIb)

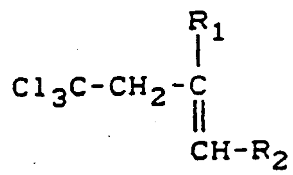 (IIIc)

in which
$R_1$ and $R_2$ have the meaning indicated under formula (I), in the presence of Friedel-Crafts catalysts.

**2.** Process according to Claim 1, characterized in that the reaction is carried out in the liquid phase.

**3.** Process according to Claim 2, characterized in that the reaction is carried out at temperatures of -50 to +200 °C.

**4.** Process according to Claim 2, characterized in that the reaction is carried out in an inert organic solvent or in an excess of a liquid aromatic compound.

**5.** Process according to one of Claims 1 to 4, characterized in that, for the preparation of monomeric diarylalkanes (diarylalkanes of the formula (I) in which n is zero), aromatic compounds of the formulae (II) and aliphatic halogen compounds of the formulae (III) are employed in a molar ratio of 10 to 30:1.

**6.** Aralkyl compounds of the formulae

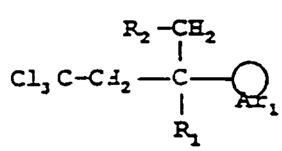 (a)

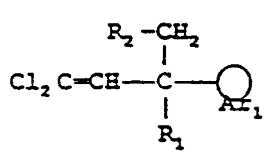 (b)

in which

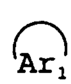

represents an aryl radical, which can be substituted by $C_1$-$C_4$-alkyl, hydroxyl, $C_1$-$C_4$-alkoxy, phenoxy, mercapto, $C_1$-$C_4$-alkylmercapto, phenylmercapto or halogen, or represents acenaphthyl,
$R_1$ represents hydrogen or a $C_1$-$C_{18}$-alkyl radical and

$R_2$ represents hydrogen or a $C_1$-$C_{18}$-alkyl radical which is optionally substituted by halogen.

**7.** Aralkyl compounds of the formulae

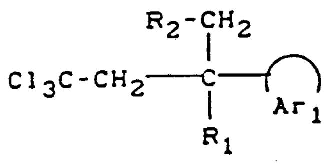 (a)

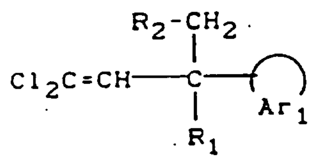 (b)

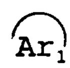

in which represents an optionally substituted aryl radical,
$R_1$ represents hydrogen or a $C_1$-$C_{18}$-alkyl radical and
$R_2$ represents hydrogen or an optionally substituted $C_1$-$C_{18}$-alkyl radical.

**8.** Aralkyl compounds according to Claim 7, characterized in that
$R_1$ represents hydrogen, methyl, ethyl, n-propyl or i-propyl and
$R_2$ represents hydrogen or methyl.

## Revendications

**1.** Procédé pour la préparation de diarylalcanes géminaux monomères ou oligomères répondant à la formule

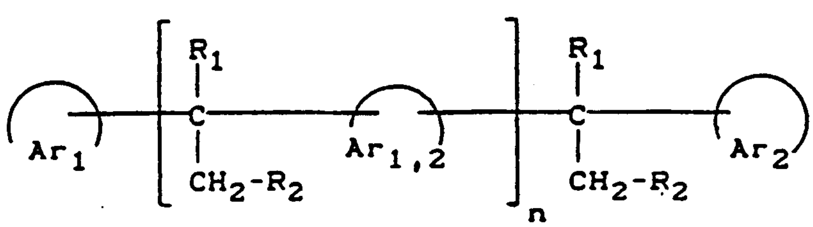 (I),

dans laquelle
n représente un nombre entier de 1 à 5 ou bien est égal à zéro

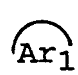 et 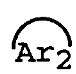

représentent, indépendamment l'un de l'autre, un radical aryle portant éventuellement un ou plusieurs substituants identiques ou différents, et

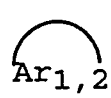

représente soit un radical

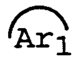,

soit un radical

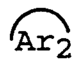,

$R_1$ représente un atome d'hydrogène ou un radical alkyle en $C_1$-$C_{18}$, et

$R_2$ représente un atome d'hydrogène ou un radical alkyle en en $C_1$-$C_{18}$ portant éventuellement un ou plusieurs substituants identiques ou différents,

caractérisé en ce qu'on fait réagir des radicaux aromatiques répondant aux formules

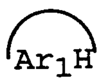 et/ou 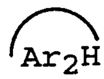 (II),

dans lesquelles

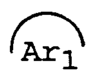 et 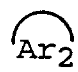

ont la signification indiquée dans la formule (I), avec un composé aliphatique halogéné répondant aux formules

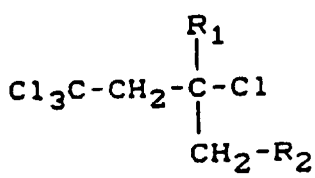 (IIIa),

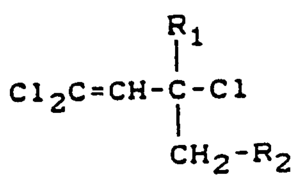 (IIIb),

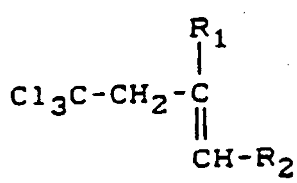 (IIIc),

dans lesquelles $R_1$ et $R_2$ ont la signification indiquée dans la formule (I), en présence de catalyseurs de Friedel-Crafts.

**2.** Procédé selon la revendication 1, caractérisé en ce qu'on procède à la mise en réaction en phase liquide.

**3.** Procédé selon la revendication 2, caractérisé en ce qu'on procède à la mise en réaction à des températures de -50 à +200 °C.

4. Procédé selon la revendication 2, caractérisé en ce qu'on procède à la mise en réaction dans un solvant organique inerte ou dans un excès du composé aromatique liquide.

5. Procédé selon l'une quelconque des revendications 1 à 4, caractérisé en ce que, pour la préparation de diarylalcanes monomères (diarylalcanes de formule (I) dans laquelle n est égal à zéro), on met en oeuvre des composés aromatiques répondant aux formules (II) et des composés halogénés aliphatiques répondant aux formules (III), dans un rapport molaire de 10 à 30:1.

6. Composés aralkyle répondant aux formules

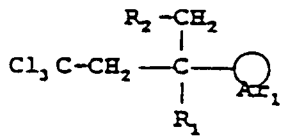

(a)

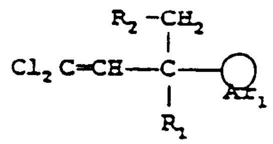

(b)

dans lesquelles

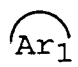

représente un radical aryle portant éventuellement un ou plusieurs substituants identiques ou différents alkyle en $C_1$-$C_4$, hydroxyle, alcoxy en $C_1$-$C_4$, phénoxy, mercapto, alkyl(en $C_1$-$C_4$)mercapto, phénylmercapto, halogéno, ou encore un groupe acénaphtyle,
$R_1$ représente un atome d'hydrogène ou un groupe alkyle en $C_1$-$C_4$,
$R_2$ représente un atome d'hydrogène ou un radical alkyle en $C_1$-$C_{18}$ portant éventuellement un ou plusieurs substituants halogéno identiques ou différents.

7. Composés aralkyle répondant aux formules

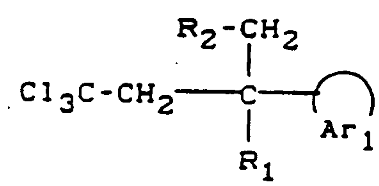

(a)

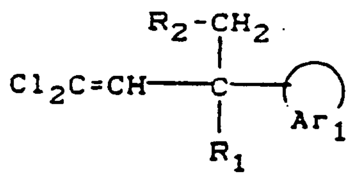

(b)

dans lesquelles

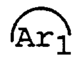

représente un radical aryle portant éventuellement un ou plusieurs substituants identiques ou différents,
$R_1$ représente un atome d'hydrogène ou un radical alkyle en $C_1$-$C_{18}$,
$R_2$ représente un atome d'hydrogène ou un radical alkyle en $C_1$-$C_{18}$ portant éventuellement un ou plusieurs substituants identiques ou différents.

**8.** Composés aralkyle selon la revendication 7, caractérisés en ce que
$R_1$ représente un atome d'hydrogène, un groupe méthyle, un groupe éthyle, un groupe n-propyle ou un groupe i-propyle, et
$R_2$ représente un atome d'hydrogène ou un groupe méthyle.